(19) Europäisches Patentamt

European Patent Office

Office européen des brevets

(11) **EP 1 084 094 B1**

(12) **EUROPÄISCHE PATENTSCHRIFT**

(45) Veröffentlichungstag und Bekanntmachung des Hinweises auf die Patenterteilung:
**30.07.2003 Patentblatt 2003/31**

(21) Anmeldenummer: **99936298.1**

(22) Anmeldetag: **21.05.1999**

(51) Int Cl.$^7$: **C07C 45/50**, C07C 47/02

(86) Internationale Anmeldenummer:
**PCT/DE99/01521**

(87) Internationale Veröffentlichungsnummer:
**WO 99/061401 (02.12.1999 Gazette 1999/48)**

(54) **VERFAHREN ZUR KATALYTISCHEN HYDROFORMYLIERUNG VON OLEFINEN IN EINER MIKROEMULSION**

METHOD FOR THE CATALYTIC HYDROFORMYLATION OF OLEFINS IN A MICROEMULSION

PROCEDE D'HYDROFORMYLATION CATALYTIQUE D'OLEFINES DANS UNE MICROEMULSION

(84) Benannte Vertragsstaaten:
**AT BE DE DK ES FR GB IT NL SE**

(30) Priorität: **25.05.1998 DE 19822968**

(43) Veröffentlichungstag der Anmeldung:
**21.03.2001 Patentblatt 2001/12**

(73) Patentinhaber: **SASOL Germany GmbH
22297 Hamburg (DE)**

(72) Erfinder:
• **SCHOMÄCKER, Reinhard
D-13589 Berlin (DE)**
• **HAUMANN, Marco
D-10437 Berlin (DE)**
• **KOCH, Herbert
D-46282 Dorsten (DE)**

(74) Vertreter: **Schupfner, Georg U.
Müller, Schupfner & Gauger,
Parkstrasse 1
21244 Buchholz (DE)**

(56) Entgegenhaltungen:
**EP-A- 0 380 154        US-A- 4 399 312**

**Beschreibung**

Gegenstand der Erfindung ist ein Verfahren zur katalytischen Hydroformylierung von Olefinen mit Kohlenmonoxid und Wasserstoff, wobei die Hydroformylierungsreaktion in einer Mikroemulsion durchgeführt wird.

1938 untersuchte Otto Roelen in den Labors der Ruhrchemie AG, Oberhausen, die Auswirkungen von Olefinen, speziell Ethylen, auf die Fischer-Tropsch-Synthese. Kleine Mengen Propanol und Diethylketon deutete Roelen als Produkte einer neuen chemischen Reaktion, der Hydroformylierung (DE 849 548, US 2 237 066).

Mitte der 50er Jahre erlangte diese jetzt als Oxo-Synthese an einem Übergangsmetall-Katalysator bekannte Umwandlung eines Alkens in einen n-Aldeyhd sowie den isomeren iso-Aldehyd steigende wirtschaftliche Bedeutung.

Zwei Faktoren waren hierfür maßgeblich verantwortlich: zum einen ermöglichten Innovationen in der petrochemischen Industrie die Herstellung der Olefine in ausreichender Menge bei gleichbleibend hoher Qualität und zu einem günstigen Preis, zum anderen wurden die Produkte der Hydroformylierung als Zwischenprodukte bei der Herstellung von PVC und Detergentien benötigt, zwei rapide wachsenden Märkten, welche auch heute noch die beiden Hauptabnehmer der Hydroformylierungsprodukte sind.

Die Katalysatoren der ersten Generation beinhalteten ausschließlich Cobalt als Katalysatormetall (BASF, ICI, Ruhrchemie). Aufgrund der hohen Stabilität der Cobaltcarbonyle waren drastische Reaktionsbedingungen erforderlich: Drücke von 200 bis 300 bar, Temperaturen von 150 °C bis 180 °C (H. Bahrmann, H. Bach, Oxo Synthesis in Ullmann, 5th ed , Vol. A 18, 321).

Der Shell-Prozeß benutzte Mitte der 60er Jahre erstmals Phosphin-Liganden anstelle des Kohlenmonoxids. Dadurch konnte das n/iso-Verhältnis gesteigert werden. Ferner waren die Reaktionsbedingungen deutlich milder.

Die zweite Katalysatorgeneration kombinierte diese Ligandenmodifizierung mit dem Austausch des Zentralatoms von Cobalt durch Rhodium. Modifizierte Rhodium-Katalysatoren erlaubten weit mildere Reaktionsbedingungen, erzielten höhere n/iso-Raten und verringerten die Hydrierung des Alkens als unerwünschte Nebenreaktion. Diese als Low-Pressure-Oxo-(LPO)-Prozesse bekannten Verfahren wurden Anfang der 80er Jahre durch die dritte Katalysatorgeneration abgelöst. Wasserlösliche Phosphinliganden ermöglichten im Ruhrchemie-Rhone-Poulenc-(RCH/RP)-Verfahren eine nahezu verlustfreie Rückgewinnung des sehr teuren Rhodiums durch Phasentrennung der wäßrigen Katalysatorlösung von der organischen Produktphase (DE-A-32 34 701, DE-A- 32 35 030).

Die Wasserlöslichkeit des Katalysators wird durch den Einbau einer oder mehrerer stark polarer Gruppen wie z.B. $-SO_3H$, $-COOH$, $-NH_2$ bzw. deren Salze in den Phosphinliganden erreicht. Die Entwicklung der Zweiphasenkatalyse ist daher eng mit der Synthese solcher Liganden verbunden.

Sulfonierte Phenylphosphine lösen sich z.B. bei jedem pH-Wert in wäßrigem Medium, carboxylierte Phosphine nur in saurem Medium. Der hydrophile Charakter des Triphenylphosphins TPP nimmt mit steigender Anzahl von Sulfonsäuregruppen zu

TPPMS <TPPDS < TPPTS = Triphenylphosphintrisulfonsäure

(TPPMS = Triphenylphosphinmonosulfonsäure, TPPDS = Triphenylphosphindisulfonsaure)

Für die industrielle Anwendung ist TPPTS der ideale Ligand für die Modifikation der Rhodiumcarbonyl-Spezies $HRh(CO)_4$, Ohne größeren synthetischen Aufwand können 3 der 4 CO-Liganden durch das leicht in. Wasser lösliche, nicht toxische TPPTS ersetzt werden.

Es ist somit möglich, maßgeschneiderte Katalysatorkomplexe für verschiedenste Anwendungen bereitzustellen.

Der größte Vorteil wasserlöslicher Liganden besteht in der leichten Abtrennung der Hydroformylierungsprodukte vom Katalysator. Üblicherweise werden homogene Katalysatoren durch thermische Trennverfahren wie Destillation/Rektifikation vom Produkt abgetrennt. Durch diese thermische Beanspruchung kann es zu Deaktivierung oder gar zum Zerfall des Katalysatorkomplexes kommen. Weiterhin ist eine verlustfreie Rückgewinnung nicht immer möglich. Im Zweiphasensystem des RCH/RP-Verfahrens liegen die Rhodiumverluste im ppb-Bereich.

Bei der Reaktion von Synthesegas mit Olefinen kann es neben der eigentlichen Hydroformylierung zu einer Reihe von Nebenreaktionen kommen, die Umsatz und Selektivität beeinflussen. Die Hydrierung der olefinischen Doppelbindung führt in einer irreversiblen Reaktion zum gesättigten Kohlenwasserstoff und wird ebenfalls durch Cobalt und Rhodium katalysiert Diese Parallelreaktion vermindert die Ausbeute an Aldehyd. Für unmodifizierte Cobalt-Katalysatoren liegt die Hydrierung in der Größenordnung von 15 % bezogen auf die Olefinmenge. Bei modifizierten Rhodium-Katalysatoren liegt diese Reaktion unter 5 %. Weitere Folgereaktionen der gebildeten Aldehyde wie Kondensation, Trimerisierung und Aldolreaktion führen zur Bildung sogenannter "heavy-end"-Produkte mit deutlich höheren Siedepunkten als die gewünschten Produkte.

Einer der Hauptnachteile der homogenen Katalyse kann durch die Zweiphasenkatalyse umgangen werden Diese Reaktionstechnik erfordert jedoch eine minimale Löslichkeit der Olefine in Wasser, ist daher auf kurzkettige Olefine beschränkt. Längerkettige Olefine können mit diesem Verfahren nicht umgesetzt werden, diese werden weiterhin homogen im Hochdruck-Verfahren (Exxon-Prozeß) an Cobaltkatalysatoren hydroformyliert.

Eine Möglichkeil zur Umsetzung von längerkettigen Olefinen nach der Zweiphasenkatalyse an Rhodiumkomplexen ist der Zusatz von Phasentransfer-Katalysaloren oder Tensiden zur wäßrigen Katalysatorlösung. Der Tensidzusatz bewirkt eine mizellare Solubilisation der wasserunlöslichen Substrate. In diesem Zusammenhang soll der Zusatz von kationischen Tensiden bevorzugt sein, da Kationtensid-Mizellen nicht nur das wasserlösliche Olefin solubillsieren, sondern über ihre positive Ladung für eine Anreicherung der negativ geladenen Rhodiumkomplex-Ionen sorgen und damit deren Anreicherung in der mizellaren Umgebung bewirken (Journal of Molecular Catalysis A: Chemical 101 1995, 179-186).

In diesem Zusammenhang werden auch sog. Tensidphosphane eingesetzt, in denen die Funktionen des Tensids für die mizellare Cokatalyse und des Komplexliganden für die Katalyse der Carbonylierungsreaktion vereinigt sind (Journal of Molecular Catalysis 66, 1991, 143-154).

Die US-A-4,399,312 offenbart die Durchführung einer Hydroformylierungsreaktion in einem zweiphasigen System (wäßrige Phase/organische Phase) ohne Ausbildung von Mikroemulsionen. Der Zusatz von geringen Mengen amphiphilen Reagenzien, wobei ionische und nichtionische Tenside mit hohen HLB-Werten genannt sind, die sehr wasserlöslich und wenig öllöslich sind, bewirkt hier einen beschleunigten Stofftransport aber nicht die Ausbildung einer Mikroemulsion.

Dennoch sind die Resultate bei Einsatz von Phasentransferkatalysatoren oder Tensiden im Hinblick auf die Reaktionsbedingungen sowie die Ausbeute noch nicht zufriedenstellend.

Ein neuer Lösungsansatz für die Hydroformylierung lankettiger Alkohole stellt die gezielte Umsetzung in Mikroemulsionen dar.

Mikroemulaionen sind ternäre Gemische aus Wasser, Öl und Tensid. Die wäßrige Phase, welche den Katalysator enthält, liegt in Tröpfchenform dispergiert in der Ölphase, dem Olefin, vor. An der Phasengrenze treffen Katalysator, Olefin und gelöstes Synthesegas zusammen und reagieren zum Aldehyd.

In der EP-A-0 380 154 wird ein Hydroformylierungsverfahren beschrieben, bei dem ein Olefin mit Wasserstoff und Kohlenmonoxid in Gegenwart eines wasserlöslichen Hydroformylierungskatalysators in einer Mikroemulsion umgesetzt wird. Die Mikroemulsion besteht aus einer Ölphase, gebildet aus einem Olefin oder aus einem Olefin und seinen Hydroformylierungsprodukten, einer wäßrigen Phase, gebildet aus dem wasserlöslichen Komplexkatalysator, einem grenzflächenaktiven Mittel und einem co-grenzflächenaktiven Mittel, wobei die Ölphase die äußere Phase ausbildet und die wäßrige Phase die innere Phase der Mikroemulsion ausbildet. Bei den zur Bildung der Mikroemulsion zwingend notwendigen co-grenzenflächenaktiven Mitteln handelt es sich bevorzugt um einwertige aliphatische Alkohole mit 3 bis 7 Kohlenstoffatomen wie insbesondere n-Butanol oder n-Pentanol. Als bevorzugte grenzflächenaktive Mittel werden anionische Tenside, wie z.B. Alkylbenzolsulfonate oder Fettalkoholsulfate eingesetzt.

Ein deutlicher Nachteil dieses Verfahrens ist der zwingend erforderliche Zusatz eines Cotensids. Dabei ist es notwendig, den Gehalt des Cotensids zu kontrollieren, da dieser für die Bildung der gewünschten Mikroemulsion verantwortlich ist, die nach Möglichkeit bis zu Umsetzungsgraden von 70 % stabil sein soll. Ein weiteres Problem ist der Befund, daß sich das co-grenzflächenaktive Mittel zum Teil in der Produktphase anreichert. Da bevorzugterweise zwischen 15 und 30 Gew.% an cogrenzflächenaktivem Mittel bei der Hydroformylierungsreaktion eingesetzt werden, kann dies zur Anreicherung erheblicher Mengen dieses Agenz in der Produktphase führen Dieser Umstand ist unerwünscht, da die bevorzugterweise als Cotensid eingesetzten kurzkettigen Alkohole aufgrund ihres Geruchs stören, z.Tl. unerwünschte Nebenreaktionen eingehen können (z.B. Acetalisierung mit den gebildeten Aldehyden) und letztendlich die Produktqualität der gewonnenen langkettigen Hydroformylierungsprodukte negativ beeinflussen

Aus diesem Grunde ist es zwingend erforderlich das Produktgemisch weiter aufzuarbeiten. Typische Aufarbeitungsschritte sind z.B. die destillative Entfernung der störenden cogrenzflächenaktiven Mittel, wie z.B. Butanol, Pentanol etc. aus dem Produktgemisch sowie die saure Spaltung der aus Cotensid und Aldehyd gebildeten Acetale.

Aufgabe der vorliegenden Erfindung war es daher, ein verbessertes Verfahren zur Verfügung zu stellen, daß frei von den geschilderten Nachteilen ist.

Überraschenderweise wurde gefunden,daß bei Einsatz nichtionischer Tenside auf den Einsatz der in der EP 0 380 154 genannten co-grenzflächen-aktiven Substanzen verzichtet werden kann und stabile Mikroemulsionen erhalten werden.

Diese Aufgabe wird daher erfindungsgemäß gelöst mit einem Verfahren zur katalytischen Hydroformylierung von Olefinen durch Umsetzung eines Olefins mit Wasserstoff und Kohlenmonoxid in einem flüssigen, wäßrig-organischen Medium in Anwesenheit eines wasserlöslichen Hydroformylierungskatalysators, dadurch gekennzeichnet, daß das wäßrig-organische Medium während eines wesentlichen Teiles der Hydroformylierungsreaktion in Form einer Mikro-emulsion vorliegt, die aus einer Ölphase, enthaltend das Olefin oder das Olefin und seine Hydroformylierungsprodukte, und einer wäßrigen Phase, enthaltend den wasserlöslichen Komplexkatalysator, und einem nichtionischen grenzflächenaktiven Mittel, gebildet wird. Auf den Zusalz eines co-grenzflächenaktiven Mittels laut EP 0 390 154 wird ausdrücklich verzichtet.

Ein großer Vorteil gegenüber dem im Stand der Technik beschriebenen Verfahren ist die Tatsache, daß die Produktphase in diesem Fall nicht destillativ aufgearbeitet werden muß, um das Cotensid zu entfernen, und die Kontrolle der Cotensidkonzentration, die maßgeblich für die Bildung der Mikroemulsion verantwortlich ist, entfällt.

In der EP 0 380 154 werden als Cotenside monohydroxy, aliphatische Alkohole mit 3 bis 7 Kohlenstoffatomen genannt.

Nach einer weiteren Ausgestaltung der Erfindung ist das Verfahren zur katalytischen Hydroformylierung von Olefinen in einem flüssigen, wäßrig - organischen Medium in Anwesenheit eines wasserlöslichen Hydroformylierungskatalysators, dadurch gekennzeichnet, daß das wäßrig-organische Medium während eines wesentlichen Teiles der Hydroformylierungsreaktion in Form einer Mikroemulsion vorliegt, die aus einer Ölphase, enthaltend das Olefin oder das Olefin und seine Hydroformylierungsprodukte, und einer wäßrigen Phase, enthaltend den wasserlöslichen Komplexkatalysator, und einem oder mehreren nichtionischen grenzflächenaktiven Mittel gebildet wird, wobei zumindest eines der nichtionischen grenzflächenaktiven Mittel ein alkoxylierter Fettalkohol ist. Bezogen auf die Gesamtzusammensetzung wird das nichtionische grenzflächenaktive Mittel zu 5 bis 30 Gew.% eingesetzt.

Der erfindungsgemäß eingesetzte alkoxylierte Fettalkohol weist, bezogen auf den Fettalkohol, vorzugsweise 7 bis 31, besonders bevorzugt 9 bis 31 Kohlenstoffatome. insbesondere 9 bis 18 Kohlenstoffatome auf.

Die alkoxylierten Fettalkohole können nach einer weiteren Ausgestaltung alkylverschlossene Alkoxylat-Endgruppen und/oder 2 bis 20 Alkoxy-, vorzugsweise Ethoxy-, Einheiten aufweisen. Vorzugsweise sind die alkoxylierten Fettalkohole engverteilt. Solche Produkte sind z.B. als Marlipal ® 013 Produkte (Produkt der Condea Chemie GmbH) im Handel. Das nichtionische grenzflächenaktive Mittel ist vorzugsweise zu mindestens 50 Gew.% ein oben bezeichneter alkoxylierer Fettalkohol.

Insbesondere ist auch der Einsatz von nichtionischen grenzflächenaktiven Mitteln, die aus dem bei der Hydroformylierungsreaktion gebildeten Aldehyd in nachfolgenden Schritten hergestellt werden können, vorteilhaft. Beispielsweise empfiehlt sich bei der Hydroformylierungsreaktion von Tributen der Einsatz eines O13-Ethoxylates, da die Hydroformylierung von Tributen einen C13-Aldehyd ergibt, der durch Hydrierung und Ethoxylierung in ein O13-Ethoxylat überführt wird. Vorteil dieser Tensidauswahl ist die Tatsache, daß durchaus ein Teil des Tensids als Verunreinigung im Hydroformylierungsprodukt verbleiben kann Das gewonnene Produkt erfährt keine Qualitätsverluste für den Fall, daß noch gewisse Mengen an nichtionischem Tensid im Produktgemisch der Hydroformylierungsreaktion enthalten sind.

Weiterhin wurde überraschenderweise festgestellt, daß die Durchführung der Reaktion nicht zwingend notwendig in einer einphasigen Mikroemulsion durchgeführt werden muß, sondern daß unter Beibehaltung der Ausbeuten und Selektivitäten die Hydroformylierung auch in einem zweiphasigen System bestehend aus einer Mikroemulsion, die im Kontakt mit einer Olefinphase steht, welche als Reservoir dient, durchgeführt werden kann. Entscheidend für die Reaktionsgeschwindigkeit scheint nur eine ausreichende Olefinkonzentration an der inneren Phasengrenzfläche der Mikroemulsion zu sein. Diese spezielle Variante der Reaktionsführung beinhaltet weitergehende Vorteile. Das System ermöglicht die Phasentrennung und Katalysatorrückführung ohne Änderung der Temperatur. Weiterhin ist das Zweiphasengebiet Mikroemulsion/Olefinphase über einen längeren Teil der Hydroformylierungsreaktionszeit existent

In einer bevorzugten Ausführungsform werden die nach dem erfindungsgemäßen Verfahren hergestellten Produkte zur Abtrennung von geringen Mengen an Rhodium bzw. Rhodiumkomplexen einer Ultrafiltration unterworfen. Durch diesen Prozeß kann der Verlust an Edelmetall weiterhin reduziert werden.

Als Olefine werden lineare oder verzweigte Olefine mit 2 bis 30, insbesondere 6 bis 30 und besonders bevorzugt Olefine mit 8 bis 12 Kohlenstoffatomen eingesetzt. Die olefinische Doppelbindung kann endständig (α-Olefine) oder auch innenständig sein.

Als Katalysatoren werden im beanspruchten Verfahren vorzugsweise Rhodiumverbindungen eingesetzt, die in komplexer Bindung wasserlösliche Phosphine enthalten, d.h. Salze, deren Anion ein Phosphin ist, das mindestens einen sulfonierten oder carboxylierten Rest enthält. Besonders bevorzugte Komplexliganden sind Phosphine mit aromatischen Resten, insbesondere sulfonierte Triarylphosphine. Generell können neben Rhodiumverbindungen auch sonstige in der Hydroformylierung aktive Metallverbindungen der Übergangsmetallreihe ausgewählt werden, wie z.B. Kobalt, Ruthenium, Osmium, Iridium, Palladium und Platin.

Die Herstellung der Hydroformylierungskatalysatoren erfolgt durch Zusammengeben der Übergangsmetalle bzw deren Verbindungen (z.B. Komplexe) mit den wasserlöslichen Phosphinliganden in wäßrigem Milieu. Die wasserlöslichen Phosphinliganden sollten im Überschuß eingesetzt werden, d.h. deutlich über dem stöchiometrischen Anteil liegen. Grundsätzlich kann der Katalysator dem Reaktionssystem auch präformiert zugesetzt werden

Das bei der Hydroformylierung eingesetzte nichtionische grenzflächenaktive Mittel ist vorzugsweise ausgewählt aus der Gruppe der alkoxylierten, insbesondere ethoxilierten Fettalkohole, alkoxylierten Alkylphenole, Fettsäuren bzw. Fettsäureester, Fette, Öle und Wachse.

Die Hydroformylierungsreaktion kann entweder kontinuierlich oder auch diskontinuierlich durchgefuhrt werden. Bevorzugt wird das erfindungsgemäße Verfahren bei Reaktionstemperaturen im Bereich von 40 bis 150 °C und Reaktionsdrücken von 2 bis 150 bar durchgeführt.

Bevorzugterweise wird die Reaktion durchgeführt bei Temperaturen von 50 bis 100 °C und Drucken von 20 bis 60 bar. Das molare Einsatzstoffverhältnis von Wasserstoff zu Kohlenmonoxid kann in weiten Grenzen variiert werden. Im allgemeinen setzt man Synthesegas ein, in dem das Volumenverhältnis von Kohlenmonoxid zu Wasserstoff Werte von 1:1 bis 1:1,5 annimmt.

Beispiel 1.

22,7 g n-1-Dodecen (Sigma-Aldrich) wurden bei Raumtemperatur mit 2,3 g einer wäßrigen Lösung versetzt, welche 44 g/l Tris-(3-sulfophenyl)-phosphin Trinatriumsalz (Sigma-Aldrich) und 5 g/l Di-μ-chloro-bis-[(cycloocta-1c,5c-dien)-rhodium(I)] enthielt. Zu diesem zweiphasigen System wurden 3,8 g Marlipal ® 013/70 (CONDEA Chemie GmbH, C13-Oxoalkoholpolyethylenglykolether (7 EO)) gegeben. Die Bildung einer Mikroemulsion wurde durch Untersuchung des Phasenverhaltens in einem Temperaturbad sichergestellt. Die so erhaltene Mikroemulsion hat folgende Zusammensetzung (in Massenprozent):

| n-1-Dodecen | 79% |
| Marlipal 013/70 | 13% |
| Wässrige Katalysator-Lösung | 8% |

Die Mikroemulsion wurde in einen 100 ml Laborautoklav überführt und auf 75°C aufgeheizt. Der Synthesegasdruck wurde auf 30 bar erhöht. Das Synthesegas hatte eine Zusammensetzung von Wasserstoff zu Kohlenmonoxid von 1:1. Das Reaktionsgemisch wurde bei diesen Bedingungen 10 Stunden gerührt. Anschließend wurde der Autoklav abgekühlt, die Reaktionslösung entspannt und dem Autoklav entnommen. Das Gemisch trennte sich in zwei Phasen, eine obere, ölreiche Phase, welche neben n-Tridecanal, iso-2-Methyldodecanal sowie nicht umgesetztes n-1-Dodecen enthält, und eine untere, wäßrige Phase, welche den Katalysator und den Großteil des Tensids enthielt. Mittels gaschromatographischer Analysen wurde ein Olefinumsatz von 61 % und ein Umsatz zum Aldehyd (sowohl n- als auch iso-Aldehyd) von 61 % bestimmt. Daraus ergab sich eine Selektivität bezüglich Aldehydbildung von 98,3 %. Das Verhältnis von gebildetem n-Aldehyd zu iso-Aldehyd betrug 2,74.

Beispiel 2:

Bei einem Synthesegasdruck von 50 bar wurde der Versuch unter sonst gleichen Bedingungen wie in Beispiel 1 durchgeführt
Nach 10 Stunden Reaktionsdauer ergab sich ein Olefinumsatz von 77,6 % und ein Umsatz zum Aldehyd von 74,7 %. Daraus errechnet sich eine Selektivität bezüglich der Aldehydbildung von 96,2 % Das n/iso-Verhältnis betrug 1,73.

Beispiel 3:

Zur Katalysator-Herstellung wurde als Rhodiumsalz $Rh(CO)_2(acac)$ verwendet. Die Umsetzung erfolgte in Analogie zu Beispiel 1 bei einem Synthesegasdruck von 100 bar. Nach 10 Stunden Reaktionsdauer ergab sich ein Olefinumsatz von 90,5 % und ein Umsatz zum Aldehyd von 90,3 %. Daraus berechnet sich eine Selektivität bezüglich der Aldehydbildung von 99,8 %. Das n/iso-Verhältnis betrug 2,3.

Beispiel 4:

Unter gleichen Bedingungen wie in Beispiel 1 wurde Tributen (technisches Isomerengemisch) bei 80 bar Synthesegasdruck und 75°C umgesetzt. Nach 10 Stunden Reaktionsdauer ergab die gaschromatographische Analyse einen Olefinumsatz von 69,6 %. Eine Bestimmung der Selektivität konnte aufgrund der großen Isomerenanzahl nicht erfolgen.

**Patentansprüche**

1. Verfahren zur katalytischen Hydroformylierung von Olefinen in einem flüssigen, wäßrig - organischen Medium in Anwesenheit eines wasserlöslichen Hydroformylierungskatalysators, **dadurch gekennzeichnet, daß**

   - das wäßrig-organische Medium während eines wesentlichen Teiles der Hydroformylierungsreaktion in Form einer Mikroemulsion vorliegt,
die aus einer Ölphase, enthaltend das Olefin oder das Olefin und seine Hydroformylierungsprodukte, und einer wäßrigen Phase, enthaltend den wasserlöslichen Komplexkatalysator, und einem oder mehreren nichtionischen grenzflächenaktiven Mitteln gebildet wird,

- wobei auf den Zusatz eines monohydroxy aliphatischen Alkohols mit 3 bis 7 Kohlenstoffatomen verzichtet wird und
- bezogen auf die Gesamtzusammensetzung 5 bis 30 Gew.% nichtionisches grenzflächenaktives Mittel eingesetzt wird.

2. Verfahren nach Anspruch 1, **dadurch gekennzeichnet, daß**

- auf den Zusatz eines co-grenzflächen-aktiven Mittels ausdrücklich verzichtet wird.

3. Verfahren zur katalytischen Hydroformylierung von Olefinen in einem flüssigen, wäßrig - organischen Medium in Anwesenheit eines wasserlöslichen Hydroformylierungskatalysators, **dadurch gekennzeichnet, daß**

- das wäßrig-organische Medium während eines wesentlichen Teiles der Hydroformylierungsreaktion in Form einer Mikroemulsion vorliegt,
die aus einer Ölphase, enthaltend das Olefin oder das Olefin und seine Hydroformylierungsprodukte, und einer wäßrigen Phase, enthaltend den wasserlöslichen Komplexkatalysator, und einem oder mehreren nichtionischen grenzflächenaktiven Mittel gebildet wird,
- wobei zumindest eines der nichtionischen grenzflächenaktiven Mittel ein alkoxylierter Fettalkohol ist und
- bezogen auf die Gesamtzusammensetzung 5 bis 30 Gew.% nichtionisches grenzflächenaktives Mittel eingesetzt wird.

4. Verfahren nach Anspruch 3, **dadurch gekennzeichnet, daß** der alkoxylierte Fettalkohol ein alkoxylierter Fettalkohol mit 7 bis 31 Kohlenstoffatomen, insbesondere mit 9 bis 31 Kohlenstoffatomen, bezogen auf den Fettalkohol, ist.

5. Verfahren nach einem der vorhergehenden Ansprüche, **dadurch gekennzeichnet, daß** das wäßrig-organische Reaktionsmedium während eines wesentlichen Teiles der Hydroformylierungsreaktion als zweiphasiges Gemisch, bestehend aus einer Mikroemulsion, die im Gleichgewicht mit einer olefinreichen Phase steht, vorliegt.

6. Verfahren nach einem der vorhergehenden Ansprüche, **dadurch gekennzeichnet, daß** die in der Produktphase enthaltenen Anteile an Hydroformylierungskatalysator durch Ultrafiltration abgetrennt werden.

7. Verfahren nach einem der vorhergehenden Ansprüche, **dadurch gekennzeichnet, daß** die Ölphase ein lineares oder verzweigtes Olefin, ausgewählt aus der Gruppe der alpha-Olefine oder der innenständigen Olefine, mit 6 bis 30 Kohlenstoffatomen, enthält.

8. Verfahren nach einem der vorhergehenden Ansprüche, **dadurch gekennzeichnet, daß** die wäßrige Phase einen wasserlöslichen Komplexkatalysator in gelöster Form enthält, der aus einem wasserlöslichen Phosphin und einem Metall, einer wasser-löslichen Verbindung oder einem wasserlöslichem Komplex eines Metalles gebildet ist, das in der Hydroformylierung aktiv ist.

9. Verfahren nach Anspruch 8, **dadurch gekennzeichnet, daß** das wasserlösliche Phosphin ein sulfoniertes Phosphin ist.

10. Verfahren nach einem der vorhergehenden Ansprüche, **dadurch gekennzeichnet, daß** das im Komplexkatalysator für die Hydroformylierung aktive Metall aus der Gruppe Cobalt, Ruthenium, Rhodium, Palladium, Osmium, Iridium und Platin ausgewählt ist.

11. Verfahren nach einem der vorhergehenden Ansprüche, **dadurch gekennzeichnet, daß** das nichtionische grenzflächenaktive Mittel unter Alkoxylaten von Fettalkoholen, Alkylphenolen. Fettsäuren bzw. Fettsäureestern, Fetten, Ölen und Wachsen ausgewählt ist.

12. Verfahren nach Anspruch 11, **dadurch gekennzeichnet, daß** das nichtionische grenzflächenaktive Mittel unter Ethoxylaten von Fettalkoholen, Alkylphenolen, Fettsäuren bzw. Fettsäureestern, Fetten, Ölen und Wachsen ausgewählt ist.

13. Verfahren nach einem der vorhergehenden Ansprüche, **dadurch gekennzeichnet, daß** das nichtionische grenzflächenaktive Mittel unter alkylverschlossenen Alkoxylaten von Fettalkoholen ausgewählt ist.

14. Verfahren nach einem der vorhergehenden Ansprüche, **dadurch gekennzeichnet, daß** das nichtionische grenzflächenaktive Mittel unter engverteilten Ethoxylaten von Fettalkoholen ausgewählt ist.

15. Verfahren nach einem der vorhergehenden Ansprüche, **dadurch gekennzeichnet, daß** zumindest eines der nichtionischen grenzflächenaktive Mittel aus dem bei der Hydroformylierungsreaktion gebildeten Aldehyd herstellbar ist.

16. Verfahren nach einem der vorhergehenden Ansprüche, **dadurch gekennzeichnet, daß** die Reaktionstemperatur im Bereich von 40 bis 150°C und der Reaktionsdruck im Bereich von 2 bis 150 bar liegt.

## Claims

1. A process for the catalytic hydroformylation of olefins in a liquid, aqueous-organic medium in the presence of a water-soluble hydroformylation catalyst, **characterized in that**

   - for a substantial period of the hydroformylation reaction, this aqueous-organic medium is present in the form of a microemulsion, which is made up of an oil phase containing the olefin or the olefin and its hydroformylation products,
     and an aqueous phase containing the water-soluble complex catalyst,
     and one or a plurality of nonionic surface-active agents,
   - wherein addition of a monohydroxy aliphatic alcohol having from 3 to 7 carbon atoms is omitted, and
   - 5 to 30 % by weight based on the total composition nonionic surface-active agents are used.

2. The process according to claim 1,
   **characterized in that** addition of a co-surfactant is expressly omitted.

3. A process for the catalytic hydroformylation of olefins in a liquid, aqueous-organic medium in the presence of a water-soluble hydroformylation catalyst, **characterized in that**

   - for a substantial period of the hydroformylation reaction, this aqueous-organic medium is present in the form of a microemulsion, which is made up of an oil phase containing the olefin or the olefin and its hydroformylation products,
   - and an aqueous phase containing the water-soluble complex catalyst,
     and one or a plurality of nonionic surface-active agents
   - wherein at least one of the nonionic surface-active agents is an alkoxylated fatty alcohol, and
   - 5 to 30% by weight based on the total composition nonionic surface-active agents are used.

4. The process according to claim 3,
   **characterized in that** the alkoxylated fatty alcohol has from 7 to 31 carbon atoms, particularly from 9 to 31, referring to the fatty alcohol.

5. A process according to any one of the preceding claims,
   **characterized in that** for a substantial period of the hydroformylation reaction, the aqueous-organic reaction medium is present as a two-phase mixture consisting of a microemulsion in equilibrium with an olefin-rich phase.

6. A process according to any one of the preceding claims,
   **characterized in that** hydroformylation catalyst moieties present in the product phase are separated by ultrafiltration.

7. A process according to any one of the preceding claims,
   **characterized in that** the oil phase contains a linear or branched olefin selected from the group consisting of alpha-olefins or internal olefins having from 6 to 30 carbon atoms.

8. A process according to any one of the preceding claims,
   **characterized in that** the aqueous phase contains a dissolved, water-soluble complex catalyst, which is made up of a water-soluble phosphine and a metal, a water-soluble compound, or a water-soluble complex of a hydroformylation-active metal.

9. The process according to claim 8,
   **characterized in that** the water-soluble phosphine is a sulfonated phosphine.

10. A process according to any one of the preceding claims,
    **characterized in that** the hydroformylation-active metal in the complex catalyst is selected from the group consisting of cobalt, ruthenium, rhodium, palladium, osmium, iridium, and platinum.

11. A process according to any one of the preceding claims,
    **characterized in that** the nonionic surface-active agent is selected from among alkoxylates of fatty alcohols, alkylphenols, fatty acids or fatty acid esters, fats, oils, and waxes.

12. The process according to claim 11,
    **characterized in that** the nonionic surface-active agent is selected from among ethoxylates of fatty alcohols, alkylphenols, fatty acids or fatty acid esters, fats, oils, and waxes.

13. A process according to any one of the preceding claims,
    **characterized in that** the nonionic surface-active agent is selected from among alkyl-terminal alkoxylates of fatty alcohols.

14. A process according to any one of the preceding claims,
    **characterized in that** the nonionic surface-active agent is selected from among narrow-distribution ethoxylates of fatty alcohols.

15. A process according to any one of the preceding claims,
    **characterized in that** at least one of the nonionic surface-active agents can be prepared from the aldehyde formed in the hydroformylation reaction.

16. A process according to any one of the preceding claims,
    **characterized in that** the reaction temperature is in the range from 40 to 150°C and the reaction pressure is in the range from 2 to 150 bar.

**Revendications**

1. Procédé d'hydroformylation catalytique d'oléfines dans un milieu liquide aqueux-organique en présence d'un catalyseur d'hydroformylation soluble à l'eau,
   **caractérisé en ce que**

   - le milieu liquide aqueux-organique est présent pendant une partie importante de la réaction d'hydroformylation sous forme d'une microémulsion, qui est constituée d'une phase huileuse, contenant l'oléfine ou l'oléfine et ses produits d'hydroformylation, et
     d'une phase aqueuse, contenant le catalyseur soluble à l'eau du complexe et d'un ou plusieurs produits tensioactifs non ioniques,

   - l'ajout d'un alcool aliphatique monohydroxy comportant 3 à 7 atomes de carbone étant abandonné et

   - par rapport à l'ensemble de la composition, 5 à 30 % en poids de produit tensioactif non ionique étant utilisés.

2. Procédé selon la revendication 1, **caractérisé en ce qu'**on abandonne expressément l'ajout d'un produit co-tensioactif.

3. Procédé d'hydroformylation catalytique d'oléfines dans un milieu liquide aqueux-organique en présence d'un catalyseur d'hydroformylation soluble à l'eau, **caractérisé en ce que**

- le milieu liquide aqueux-organique est présent pendant une partie importante de la réaction d'hydroformylation sous forme d'une microémulsion, qui est constituée d'une phase huileuse, contenant l'oléfine ou l'oléfine et ses produits d'hydroformylation, et
  d'une phase aqueuse, contenant le catalyseur soluble à l'eau du complexe et d'un ou plusieurs produits tensioactifs non ioniques,

- au moins un des produits tensioactifs non ioniques étant un alcool gras alcoxylisé et

- par rapport à l'ensemble de la composition, 5 à 30 % en poids de produit tensioactif non ionique étant utilisés.

4. Procédé selon la revendication 3, **caractérisé en ce que** l'alcool gras alcoxylisé est un alcool gras comportant 7 à 31 atomes de carbone, notamment 9 à 31 atomes de carbone, par rapport à l'alcool gras.

5. Procédé selon une des revendications précédentes, **caractérisé en ce que** le produit réactionnel aqueux-organique est présent pendant une grande partie de la réaction d'hydroformylation en tant que mélange à deux phases, consistant en une microémulsion qui est en équilibre avec une phase riche en oléfine.

6. Procédé selon une des revendications précédentes, **caractérisé en ce que** les proportions de catalyseurs d'hydroformylation contenues dans la phase de produit sont séparées par ultrafiltration.

7. Procédé selon une des revendications précédentes, **caractérisé en ce que** la phase huileuse est une oléfine linéaire ou ramifiée, sélectionnée dans le groupe des alpha-oléfines ou des oléfines internes, comportant 6 à 30 atomes de carbone.

8. Procédé selon une des revendications précédentes, **caractérisé en ce que** la phase aqueuse contient un catalyseur soluble à l'eau de complexe sous forme dissoute qui est constitué d'une phosphine soluble à l'eau et d'un métal, d'un composé soluble à l'eau ou d'un complexe soluble à l'eau d'un métal qui est actif dans l'hydroformylation.

9. Procédé selon la revendication 8, **caractérisé en ce que** la phosphine soluble à l'eau est une phosphine sulfonisée.

10. Procédé selon une des revendications précédentes, **caractérisé en ce que** sont choisis dans le catalyseur du complexe pour l'hydroformylation des métaux actifs du groupe cobalt, ruthénium, rhodium, palladium, osmium, iridium et platine.

11. Procédé selon une des revendications précédentes, **caractérisé en ce que** le produit tensioactif non ionique est choisi parmi des alcoxylates d'alcools gras, des alkylephénols, des acides gras ou esters d'acides gras, des graisses, des huiles et des cires.

12. Procédé selon la revendication 11, **caractérisé en ce que** le produit tensioactif non ionique est choisi parmi des éthoxylates d'alcools gras, des alkylephénols, des acides gras ou esters d'acides gras, des graisses, des huiles et des cires.

13. Procédé selon une des revendications précédentes, **caractérisé en ce que** le produit tensioactif non ionique est choisi parmi des alcoxylates scellés à l'alkyle d'alcools gras.

14. Procédé selon une des revendications précédentes, **caractérisé en ce que** le produit tensioactif non ionique est choisi parmi des éthoxylates intimement répartis d'alcools gras.

15. Procédé selon une des revendications précédentes, **caractérisé en ce qu'**au moins un des produits tensioactifs non ioniques peut être fabriqué à partir de l'aldéhyde formé lors de la réaction d'hydrofyrmulation.

16. Procédé selon une des revendications précédentes, **caractérisé en ce que** la température réactionnelle se situe dans une plage de 40 à 150 °C et la pression de température dans une plage de 2 à 150 bar.